# EUROPEAN PATENT APPLICATION

(11) **EP 2 620 055 A1**
(43) Date of publication of application: **31.07.2013**
(21) Application number: 12152861.6
(22) Date of filing: 27.01.2012
(51) Int. Cl.: A01K 67/027, A61L 26/00

(54) **Mouse models for osteoinduction and heterotopic ossification**

(71) Applicant: Universiteit Twente, 7522 NB Enschede (NL)
(72) Inventor: Barradas, Ana Margarida Cravo, 7522 NB Enschede (NL); de Boer, Jan, 7522 NB Enschede (NL)
(74) Representative: Jansen, Cornelis Marinus

(57) **Abstract**

The invention provides mouse models that are particularly suitable for osteoinductive research. In particular, mouse models are provided which have a high incidence of heterotopic bone formation in the presence of a subcutaneously implanted osteoinductive scaffold.

## Description

### FIELD OF THE INVENTION

The invention relates to the fields of biology, biochemistry and medicine. More specifically, the invention relates to mouse models for osteoinduction and uses thereof.

### BACKGROUND OF THE INVENTION

Serious bone defects often require orthopedic intervention, such as bone grafting. Autologous bone grafting involves replacing missing bone by bone that has been obtained from the same individual, for instance from the iliac crest. However, since the amount of bone that can be harvested is limited, other strategies are often required. One of the strategies in orthopedics to repair bone fractures that the patient's body cannot heal by itself is the use of porous ceramics, such as calcium phosphate ceramics, as grafting material. Such porous ceramics are osteoconductive, meaning that they can function as a scaffold to which new bone cells can adhere, grow and divide, thereby forming new bone. These ceramics have a similar chemical composition to natural bone and are capable, in general, to promote conduction of bone growth during the regeneration process. A class of these materials has been recognized as osteoinductive, thus adding to their osteoconductive properties the ability to induce new bone formation, or in other words, to promote osteogenesis of stem cells that surround the grafting material. The biological responses triggered by osteoinductive materials that lead to bone formation are, however, poorly known. Such knowledge could accelerate the design of bone healing strategies with great social and economical impacts since osteoinductive scaffolds are synthetic materials, often easier to obtain and at lower cost, compared to other bone graft substitutes. Knowledge about the biological pathways leading to new bone formation would aid in designing improved scaffolds and medicines to promote bone formation. On the other hand, such knowledge would allow the development of strategies and medicines for counteracting unwanted bone formation, which for instance occurs in patients that suffer from heterotopic ossification. Heterotopic ossification (HO) is a debilitating disorder in which bone forms at an undesired site, such as in the muscle or close to joints, resulting in deformation and impediment of normal movements. HO poses a serious problem because pathological bone masses cause pain, limit the range of motion, induce pressure ulcers and may damage the nerve. The initial trigger can be nerve damage, trauma, surgery or an inflammatory response, but the true mechanism is not known. Current drugs cannot effectively eliminate these excessive bone masses and typically affect normal bone as well. Traditionally, non steroidal antiinflammatory drugs (NSAIDs), bisphosphonates or radiation therapy are prescribed, but their action is far from effective.

Pre-clinical models involving large animals, such as sheep, dogs and goats, have been used to investigate osteoinductive materials in order to fine-tune their osteoinductive physico-chemical properties. However, the fact that only large animal models could be used in these studies has delayed the comprehension of the complex interactions between the host and the material. The use of such large animals involves high costs and strict ethical considerations, thereby limiting their use for screening for the osteoinductive potential of materials. Moreover, breeding of (genetically modified) large animals is more time consuming than breeding of small animals in view of their longer gestation periods and smaller numbers of offspring. Small animal models would advance the research, amongst other things due to larger availability of complementary research tools. Moreover, small animals such as mice can be bred in large numbers and are easy to handle. In view of these advantages, osteoinduction in small animals has been tested by various groups. However, small animals appeared unsuitable for osteoinductive research. For unknown reasons, osteoinductive scaffolds such as calcium phosphate ceramics that effectively promote bone generation in large animals did not - or to a very small extent - result in bone formation in small animals. For instance, Yuan et al (Tissue Engineering 12(6), 2006, 1607-1615) used hydroxyapatite (HA) scaffolds and biphasic calcium phosphate (BCP) scaffolds in order to investigate heterotopic bone formation. The scaffolds were introduced in intramuscular pockets of dogs, rabbits and rats and in subcutaneous pockets of mice. Bone formation was efficiently induced in dogs by both kinds of scaffolds. In rabbits, bone formation was observed in 6 out of 8 BCP implants, whereas the HA scaffolds did not induce any bone formation. In rats, no bone formation was obtained at all. In mice, the HA scaffolds did not induce bone formation either. Furthermore, the BCP scaffolds did not lead to bone formation in 13 out of 16 mice. Although in 3 out of 16 mice some bone formation could be detected, the amount of induced bone was smaller than 1%. Such low incidence of heterotopic bone formation with such poor yield obviously does not enable osteoinduction research. Yuan et al further describe that the reasons for the differences in osteoinduction for each animal species is not clear. Differences in the levels of bone morphogenetic proteins and stem cells at the implantation site could be involved.

Yang et al (Journal of Zhejiang University - Science B (Biomedicine & Biotechnology) 12(7), 2011: 582-590) implanted biphasic calcium phosphate ceramics (BCP) in the leg muscles of Balb/C mice. Although bone tissue was observed, it is unknown whether this is the result of *de novo* heterotopic bone formation. Intramuscularly implanted scaffolds have the disadvantage that bone tissue can evolve from surrounding bone tissue, by ingrowth of surrounding bone tissue via the blood vessels. This is unwanted in osteoinductive research, because a material's capability of inducing *de novo* heterotopic bone formation should be investigated. Only a capability of inducing *de novo* heterotopic bone formation makes a scaffold suitable as an implant for bone formation in a subject, because new bone formation should occur independently from existing bones, which may be damaged or absent or located too far away from the site of implantation of the scaffold. Moreover, heterotopic ossification research is aimed at investigating possible causes of *de novo* bone formation in muscles, and at investigating therapeutic interventions that may prevent, delay, diminish or inhibit *de novo* bone formation. Hence, also for this field of research it is of utmost importance that a model is used wherein *de novo* bone formation occurs, instead of ingrowth of surrounding bone tissue via blood vessels. Therefore, osteoinductive research with subcutaneously implanted materials is preferred because existing bone tissue cannot grow into subcutaneous scaffolds. Hence, bone formed at the implantation site of subcutaneous scaffolds has been formed independently from existing bone tissue.

In view of the poor results in the art, mice are not considered suitable animal models for osteoinductive research and heterotopic bone formation. This holds particularly true for subcutaneously implanted materials. Clearly there is a need for small animal models wherein osteoinduction can be efficiently induced and/or investigated.

It is an object of the present invention to provide such small animal models and uses thereof for osteoinductive research and heterotopic bone formation.

### SUMMARY OF THE INVENTION

The invention provides mouse models which are particularly suitable for osteoinductive research. In particular, mouse models are provided which have a high incidence of heterotopic bone formation in the presence of a subcutaneously implanted osteoinductive scaffold.

In a first aspect the present invention provides a mouse model for investigating osteoinduction, comprising a subcutaneously implanted material, wherein the mouse model is **characterized in that** the incidence of heterotopic bone-formation in the presence of a subcutaneous osteoinductive scaffold is at least 25%. In a preferred embodiment, the incidence of heterotopic bone-formation in the presence of a subcutaneous osteoinductive scaffold is at least 30%, more preferably at least 40%, more preferably at least 50%, more preferably at least 60%, more preferably at least 70% and most preferably at least 80%.

Now that a mouse model with a high incidence of heterotopic bone formation (herein also called heterotopic ossification) has been provided, it has become possible to perform efficient osteoinductive research with small laboratory animals. A use of a mouse model according to the invention for investigating osteoinduction is therefore also provided.

Another aspect provides a use of a mouse model according to the invention for obtaining heterotopic bone, or for studying the osteoinductive capacity of a test material.

Further provided is a method for inducing bone formation, the method comprising subcutaneously implanting an osteoinductive material into a mouse model wherein the incidence of heterotopic bone-formation in the presence of a subcutaneous osteoinductive scaffold is at least 25%, and allowing the formation of heterotopic bone.

In another aspect the invention provides a method for determining whether a test material is capable of osteoinduction, the method comprising subcutaneously implanting said test material into a mouse model wherein the incidence of heterotopic bone-formation in the presence of a subcutaneous osteoinductive scaffold is at least 25%, and determining whether heterotopic bone formation occurs at the site of implantation. Preferably, said method further comprises selecting a material that has osteoinductive capacity.

Again, the above mentioned incidence of heterotopic bone-formation in the presence of a subcutaneous osteoinductive scaffold is preferably at least 30%, more preferably at least 40%, more preferably at least 50%, more preferably at least 60%, more preferably at least 70% and most preferably at least 80%.

In a preferred embodiment, the material that is subcutaneously implanted in a mouse model according to the invention is a ceramic, more preferably a calcium phosphate ceramic.

Aspects of osteoinductive research include investigation of biological pathways involved in heterotopic bone formation. One preferred embodiment of a use or method according to the invention therefore comprises determining whether a genomic nucleic acid sequence or a biological pathway of said mouse model is associated with osteoinduction.

Another preferred embodiment of a use or method according to the invention comprises determining whether a candidate substance is capable of enhancing heterotopic ossification.

Another preferred embodiment of a use or method according to the invention comprises determining whether a candidate substance is capable of counteracting heterotopic ossification.

In another preferred embodiment of a mouse model, use or method according to the invention, said material is implanted in a dorsal pocket, a leg or the abdomen of said mouse.

In still another preferred embodiment of a mouse model, use or method according to the invention, said mouse model is able to form heterotopic bone in the presence of a subcutaneous osteoinductive scaffold, with an average bone area / scaffold area of at least 1% after 12 weeks. Preferably, the average bone area / scaffold area is higher than 1.5%, more preferably higher than 2.0%, most preferably higher than 2.5%.

In one embodiment, a mouse model according to the invention is an FVB mouse or a 129 mouse, or any substrain or related inbred strain or progeny thereof. More preferably, said mouse model is an FVB/N mouse or a 129S mouse. In one preferred embodiment, said mouse model is a 129S2 mouse, more preferably a 129S2/Sv mouse, even more preferably a 129S2/SvPasCrl mouse. In a particularly preferred embodiment said mouse model is a FVB/NCrl mouse.

Further provided is a mouse model, use or method according to the invention , wherein said mouse model comprises a subcutaneously implanted osteoinductive scaffold.

In yet another preferred embodiment, a mouse model, use or method according to the invention is provided, wherein said mouse model comprises heterotopic bone wherein the average bone area / scaffold area is higher than 1%. Preferably, the average bone area / scaffold area is higher than 1. 5%, more preferably higher than 2.0%, most preferably higher than 2.5%.

### BRIEF DESCRIPTION OF THE DRAWINGS

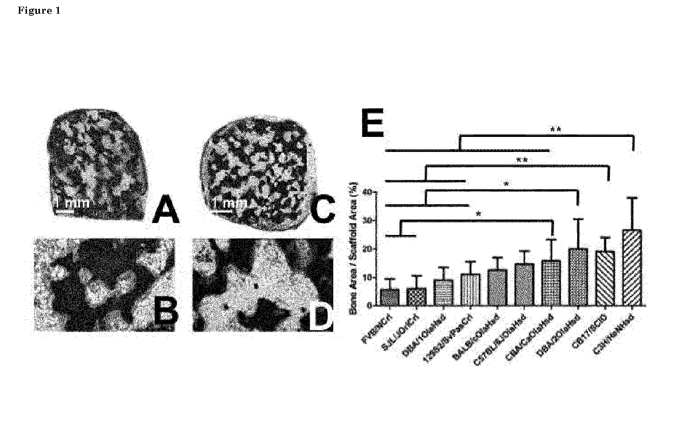

Figure 1 shows representative images of TCPb (TCP+rhBMP-2) sectioned explants from FVB/NCrl mice (A and B) and C3H/HeNHsd mice (C and D) after 12 weeks. Bone is stained bright pink and scaffold material dark brown. All TCPb explants showed bone formation, however large variations on bone area / scaffold area were observed between mice strains, ranging from approximately 6 to 25% (E). ** = *p<0.0.1;* * = *p<0.05.*

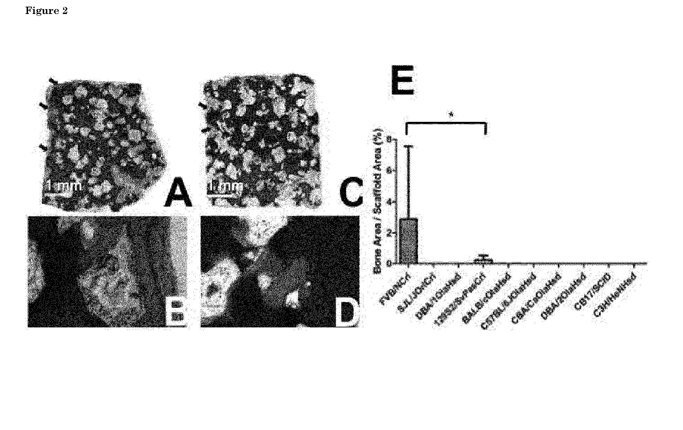

Figure 2 shows representative images of tissue of TCP sectioned explants from FVB/NCrl mice (A and B) and 129S2/SvPasCrl mice (C and D) after 12 weeks, the two strains in which bone formation was observed. Bone is stained bright pink and scaffold material dark brown. FVB/NCrl mice showed a statistically significant higher amount of bone area / scaffold area compared to FVB/NCrl (E). * *= p<0.05.*

### DETAILED DESCRIPTION OF THE INVENTION

The present invention provides mouse models with a high incidence of heterotopic bone formation in the presence of a subcutaneous osteoinductive scaffold.

Porous calcium phosphate ceramic scaffolds are currently used for heterotopic bone formation because of their known osteoinductive capabilities. However, the lack of small laboratory animal models for osteoinductive research has hampered the understanding of the process of heterotopic bone formation. Moreover, the development of improved osteoinductive scaffolds is delayed by the fact that screening at a large scale for osteoinductive materials and for substances capable of enhancing or counteracting bone formation is not practical with large animals such as dogs and goats. Heterotopic bone formation using subcutaneously implanted scaffolds in small laboratory animals such as mice was either not possible at all, or with such low incidence that no meaningful research was possible. The mouse models according to the present invention solve this problem. When an osteoinductive scaffold is implanted subcutaneously in a mouse model according to the invention, a high incidence of heterotopic bone-formation is obtained. This allows for the first time efficient osteoinductive research in mice. Osteoinductive research using a mouse model according to the present invention can be conducted easier, faster and with lower costs as compared to the currently used large animals. For instance, candidate scaffolds can be tested for osteoinductive capabilities. Alternatively, or additionally, test substances can be investigated for their capability of enhancing or counteracting osteoinduction after implantation of a scaffold with known osteoinductive properties. Moreover, the biological processes and pathways involved in osteoinduction and heterotopic bone formation can be investigated more easily and in more detail. For instance, if a certain gene or locus is suspected to be involved in heterotopic bone formation, knock out mice for such gene or locus are produced more rapidly as compared to large animals such as dogs.

Furthermore, heterotopic ossification can now be investigated more easily, because an osteoinductive scaffold implanted under the skin of an animal resulting in bone formation is a model that resembles some of the aspects that characterize the disease in a real clinical scenario. Such models existed with larger animals but now it can be applied to a mouse model according to the invention. Important applications are: 1) analysis of mechanisms behind HO using molecular and genetic research tools available for mice (basis for development of new therapies); and 2) Screening of HO inhibitory drugs while stimulus for bone formation is present (subcutaneous scaffold).

HO research with mouse models according to the present invention is preferred over current technology, for at least the following reasons. Since the biological mechanism leading to HO is not fully understood, researchers do not know which relevant parameters should be included in an *in vitro* setup. Therefore, screening for drugs to counteract HO relies on animal models. The current models are based on:
- implantation of supra-physiological concentrations of bone-inducing growth factors (mice, rats,...);
- implantation of bone marrow-derived cells (mice, rats,...);
- implantation of microporous CaP ceramic (sheep, dog, baboon);
- injection of irritants like ethanol or acid-ethanol (rabbits);
- induction of trauma by use of blunt force (sheep);
- hip arthroplasty (rabbits);
- Achilles tenotomy (rats);
- immobilization techniques (rabbits).

These models have many drawbacks. The implantation models in rodents are irrelevant for the human condition because they require supra-physiological concentrations of growth factors to induce bone. Because this is not the initial trigger for acquired HO in man, testing drugs in these models will be of limited use. The Achilles tenotomy model can also be performed in rodents (rats), but ectopic bone formation in the Achilles tendon is a rare condition in humans, again diminishing its clinical value. Models dependent on trauma, immobilization or irritants do not contemplate rodents but larger animals, increasing the research costs and again, in some cases, without relevance for the human condition (e.g. alcohol injections).

Implantation models with subcutaneously implanted scaffolds are relevant to the human condition because they combine surgery, mild trauma, nerve damage, hypercalcemia and an inflammatory response, which are all factors that have been associated with heterotopic bone formation. Until now, however, they could only be used in large animals such as sheep, dogs and baboons, which are expensive animals and require trained surgeons to perform the surgeries.

The mouse models provided by the present invention allow for the use of subcutaneously implanted osteoinductive scaffolds in mice, combining a clinically relevant setup with a cheap animal and associated surgical costs. Furthermore, it is possible to provide a scaffold stimulus that is just sufficient to elicit an answer, meaning that only relevant physiological parameters that trigger the (pathological) bone formation are awakened by it.
The use of a mouse model according to the present invention is also more ethical than most currently used HO animal models. In some of those currently used models, animals are exposed to infliction of pain and discomfort due to immobilization and/or repeated blunt force. With a mouse model according to the present invention, the level of discomfort or pain is very low, benefiting the animals.

The invention therefore provides a mouse model for investigating osteoinduction, comprising a subcutaneously implanted material, wherein the mouse model is **characterized in that** the incidence of heterotopic bone-formation in the presence of a subcutaneous osteoinductive scaffold is at least 25%. The subcutaneously implanted material may be a test material, in order to search for improved osteoinductive scaffolds. In another embodiment the subcutaneously implanted material is a material with known osteoinductive properties, for instance to search for substances that enhance or counteract heterotopic bone formation, or for obtaining heterotopic bone in sufficient quantities to investigate the biological pathways involved with its formation. A mouse model according to the invention is **characterized in that** the incidence of heterotopic bone-formation in the presence of a subcutaneous osteoinductive scaffold is at least 25%. This means that, if an osteoinductive scaffold is implanted subcutaneously in a population of a mouse model according to the invention, at least 25% of the mice of said population will exhibit heterotopic bone formation. Until the present invention such mouse model was not available. The above cited reference of Yuan et al only observed some bone formation in 3 out of 16 mice after subcutaneous implantation of an osteoinductive scaffold, which is an incidence of only 18.75%. Of course, the higher the incidence, the more suitable a mouse model according to the invention is. In the Example is shown that incidences of 80% and even 100% can be obtained. A preferred embodiment of the invention therefore provides a mouse model according to the invention, wherein the incidence of heterotopic bone-formation in the presence of a subcutaneous osteoinductive scaffold is at least 30%, more preferably at least 40%, more preferably at least 50%, more preferably at least 60%, more preferably at least 70% and most preferably at least 80%. Even more preferably, a mouse model according to the invention is provided wherein the incidence of heterotopic bone-formation in the presence of a subcutaneous osteoinductive scaffold is at least 90%, most preferably at least 95%.

As used herein, a population of a mouse model according to the invention is defined as a plurality of mice according to the invention, said plurality comprising at least two mice. Said population for instance comprises the offspring of a mouse model of the invention.

Osteoinduction means a process wherein the formation of new bone is induced by promoting osteogenesis of stem cells. The process of bone formation generally involves attachment or adhesion of bone cells to an implant/scaffold, growth of new bone tissue and deposition of new bone mineral. Bone induction generally involves a change of the cellular structure of mesenchymal tissue to become bone like.

An osteoinductive capability means the capability of a certain material or substance to promote osteogenesis of stem cells, thereby inducing bone formation.

An osteoconductive material enables bone formation by providing a structure that supports migration of newly formed bone cells, enabling them to form bone tissue. In general, osteoinductive materials have the ability to commit mesenchymal cells to bone like cells (they signal for cell differentiation).

The terms implant and scaffold are used herein interchangeably. An implant or scaffold is defined as any material suitable for the formation of new bone. Bone tissue may be formed at, on, in and/or near the implant or scaffold. Bone formation may be induced *ex vivo,* using an osteoinductive scaffold, where after the scaffold is implanted in a subject. Alternatively, an osteoinductive scaffold is implanted in a subject, where after bone formation occurs *in vivo.* In one embodiment, such scaffold is implanted at a site of interest in a subject in order to either replace or to supplement bone that is damaged, weakened or absent.

Generally, the term scaffold is used in order to emphasize that the material is particularly suitable for supporting bone growth. Preferably, the scaffold is shaped for allowing bone cells to adhere or attach to, or in, the scaffold, thereby growing new bone tissue. Preferably, a porous scaffold is used in a mouse model of the invention, meaning that the scaffold material contains empty spaces, or pores, preferably interconnected by openings or voids. Such pores support bone formation particularly well because sufficient structure is provided that supports newly formed bone tissue.

Heterotopic bone formation is defined herein as the formation of bone other than the formation and maintenance of the original skeleton of a subject. In one aspect heterotopic bone is formed *ex vivo* or at a site of a subject where normally no bone formation occurs. It is, however, also possible to form heterotopic bone at a site of a subject's original skeleton, for instance to replace or supplement a part of a subject's original skeleton bone, which may be damaged, weakened, broken or absent.

A subcutaneously implanted material is defined herein as any material that is implanted in the skin of a subject, in or below the epidermis but above the muscle. Preferably, the material is implanted in the subcutaneous tissue. At least partial implantation in the dermis or epidermis is also embraced. In the context of the present invention, a subcutaneously implanted material is preferably implanted in a dorsal pocket, a leg or the abdomen of a mouse model according to the invention.

Now that a mouse model according to the invention is provided which has a high incidence of heterotopic bone formation, osteoinduction is more easily investigated. One aspect of the invention therefore provides a use of a mouse model according to the invention for investigating osteoinduction. As discussed above, in one embodiment osteoinductive research with a mouse model according to the invention involves screening for new osteoinductive materials in order to design improved scaffolds for osteoinduction. One aspect of the invention therefore provides a use of a mouse model according to the invention for studying the osteoinductive capacity of a test material. One embodiment provides a method for determining whether a test material is capable of osteoinduction, the method comprising:
- subcutaneously implanting said test material into a mouse model wherein the incidence of heterotopic bone-formation in the presence of a subcutaneous osteoinductive scaffold is at least 25%, and
- determining whether heterotopic bone formation occurs at the site of implantation. Preferably, said test material is implanted subcutaneously into a mouse model according to the invention wherein the incidence of heterotopic bone-formation in the presence of a subcutaneous osteoinductive scaffold is at least 30%, more preferably at least 40%, more preferably at least 50%, more preferably at least 60%, more preferably at least 70%, more preferably at least 80%, more preferably at least 90%, most preferably at least 95%. Two non-limiting examples of mouse models according to the invention with incidences of at least 80% and 100% are shown in the Example.

Said test material can be any material which is considered a candidate for the production of an osteoinductive scaffold. In view of the fact that implantation of an osteoinductive scaffold in humans is an important application, a test material is preferably used that is suitable for implantation in humans. This means that the test material is preferably tolerated by the human immune system and that it is non-toxic. For instance, a ceramic is tested because ceramics are known for their osteoconductive properties, they are non-toxic and they are tolerated well in humans. Said ceramic preferably comprises a calcium phosphate ceramic. Of course, other materials such as (synthetic) polymers can be equally well tested. Porous materials are preferred.

A material is for instance tested by implanting one, or several, piece(s) of the test material subcutaneously in a mouse model according to the invention. Preferably, the test materials are implanted in a dorsal pocket, a leg or the abdomen of said mouse. The materials to be implanted can have any shape. For instance, blocks or cylinders are used. In view of the available subcutaneous space in mice, blocks with a size of between 1x1x1 mm and 8x8x8 mm, preferably between 3x3x3 mm and 6x6x6 mm, or cylinders with a diameter of between 1 and 8 mm and a length of between 1 and 8 mm are preferably used. It is emphasized, however, that the shape and size of the test materials is in no way limiting the invention, as long as it is possible to implant them subcutaneously into a mouse model according to the invention. After implantation of the test material, the mice are preferably fed *ad libitum* for a period of time that is sufficient long so that heterotopic bone is allowed to form if the test material is osteoinductive. Preferably, a period of at least 20 days, preferably at least 30 days, more preferably at least 40 days, even more preferably at least 50 days, even more preferably at least 60 days is allowed between the time of implantation and the determination whether or not bone formation has occurred at the site of implantation. Of course, longer periods of time are also possible. For practical reasons, the incubation period is preferably not too long. First, this would slow down the screening process. Second, for use as *in vivo* osteoinductive scaffolds in humans, bone formation should not be too slow. Hence, if a test material does not elicit heterotopic bone formation within a reasonable period of time, for instance within at most six moths, the test material is not considered suitable for use as a scaffold in humans. Such test material could then, however, still be suitable for other purposes, such as for instance genetic analysis.

When a test material appears to have osteoinductive properties, it is preferably selected for further use. Also provided is therefore a method for determining whether a test material is capable of osteoinduction, the method comprising:
- subcutaneously implanting said test material into a mouse model wherein the incidence of heterotopic bone-formation in the presence of a subcutaneous osteoinductive scaffold is at least 25%, preferably at least 30%, more preferably at least 40%, more preferably at least 50%, more preferably at least 60%, more preferably at least 70%, more preferably at least 80%, more preferably at least 90%, most preferably at least 95%;
- incubating for at least 20 days, preferably at least 30 days, more preferably at least 40 days, more preferably at least 50 days, more preferably at least 60 days;
- determining whether heterotopic bone formation occurs at the site of implantation; and
- selecting a material that has osteoinductive capacity. As said before, the test material is preferably implanted in a dorsal pocket, a leg or the abdomen of said mouse

The selected osteoinductive material is preferably used for further osteoinductive research and/or for the development of an improved osteoinductive scaffold. For instance, a selected material is further tested in larger animals at orthotopic sites (created bone defects) in order to confirm its osteoinductive properties, where after it is further investigated and/or developed for human use.

Besides development of new or improved osteoinductive scaffolds, a mouse model according to the present invention is particularly suitable for genetic analysis. Until now, the biological pathways involved in osteoinduction and heterotopic ossification are only poorly understood. With a mouse model according to the present invention, an efficient research tool is provided for investigating osteoinduction. In one embodiment, a scaffold with known osteoinductive capacity is implanted subcutaneously into a mouse model according to the invention and heterotopic bone is allowed to form. Samples are taken from said mouse model, preferably both before and after implantation, and nucleic acid expression levels and/or protein expression levels are measured. Preferably, several samples are taken from said mouse model at different time points after implantation. Differences in expression levels before and after implantation of the osteoinductive scaffold, and/or differences in expression levels in time after implantation, provide information about biological pathways that are involved in the formation of heterotopic bone formation. Once one or more genomic nucleic acid sequences or proteins are identified that exhibit differential expression levels, it is possible to specifically focus on these proteins or nucleic acid sequences. For instance, a knock out variant of a mouse model according to the invention for such nucleic acid sequence, or for a gene encoding such protein, is produced. As used herein, a knock out variant means an animal wherein a certain nucleic acid sequence is rendered less functional as compared to an unmodified mouse model according to the invention. Preferably, such nucleic acid sequence is rendered essentially non-functional, meaning that it is at most 25%, preferably at most 20%, more preferably at most 15 %, even more preferably at most 10% even more preferably at most 5% functional as compared to a mouse model according to the invention. Preferably, said nucleic acid is rendered functionally inactive, meaning that no activity is detectable at all. Subsequently, an osteoinductive scaffold is implanted subcutaneously into such knock out variant in order to investigate the level of heterotopic bone formation. If the level of heterotopic bone formation appears to be significantly different in such knock out variant, as compared to the level of heterotopic bone formation in the same kind of non-knock out mouse model according to the invention in the presence of the same kind of scaffold, it indicates that the nucleic acid sequence that has been at least partly inactivated is involved in (a pathway of) heterotopic bone formation. It is also possible to use a QTL mapping approach. Quantitative trait loci (QTLs) are stretches of DNA containing or linked to the genes that underlie a quantitative trait. Hence, using QTLs it is possible to look for loci in the genome that code for certain traits. This is for instance done with bioinformatics tools and amplification tools such as PCR. This approach is very potent to see where a certain trait is codified in the genome. For instance, if a QTL appears to be correlated to heterotopic ossification, the genes contained in or linked to the QTL can be investigated in more detail.

A nucleic acid sequence of a mouse model according to the invention can be rendered less functional (also called "functionally deleted" or "inactivated") by methods that are well known in the art. For instance, a genomic nucleic acid sequence of interest is inactivated by altering the sequence by site directed mutagenesis. For instance, a frame shift mutation is induced. It is also possible to alter a genomic sequence of interest using homologous recombination. In this case, an exogenous nucleic acid replaces at least part of the genomic nucleic acid of interest, so that the resulting nucleic acid sequence is less functional or not functional at all. The above mentioned techniques are preferably performed with mouse embryonic stem cells, which are subsequently used to form knock out mice using well known methods. It is also possible to functionally delete a genomic nucleic acid of interest without excising or mutating it, for instance by administration of complementary nucleic acid to the mouse model. This is for instance performed by administration of a nucleic acid molecule containing the complementary nucleic acid, such as for instance siRNA, miRNA, shRNA, etc. Upon binding of the complementary nucleic acid sequence, the nucleic acid sequence of interest is rendered less functional.

The art provides various methods for testing whether a genomic nucleic acid sequence is functionally deleted. For instance, Western Blotting or nucleic acid hybridisation methods are used to screen for knock out variants.

Instead of using genetic engineering methods, a mouse model according to the present invention can also be used for genetic analysis in alternative ways. For instance, it can be tested whether a compound with a known influence on a certain pathway is capable of influencing osteoinduction. This is for instance done by comparing the extent of heterotopic bone formation in a mouse model according to the invention which comprises a subcutaneously implanted osteoinductive scaffold and which has not been provided with said compound, to the extent of heterotopic bone formation in a mouse model according to the invention that comprises a subcutaneously implanted osteoinductive scaffold and that has been provided with said compound. If the extent of heterotopic bone formation differs significantly between mice with and mice without the compound, it is concluded that the pathway which is influenced by said compound is involved in osteoinduction.

Of course, many alternative research strategies can now be exploited with a mouse model according to the invention.

One aspect of the invention therefore provides a use or method according to the invention, comprising determining whether a genomic nucleic acid sequence or a biological pathway of a mouse model according to the invention is associated with osteoinduction.

Once it is concluded that a certain genomic nucleic acid sequence or biological pathway of a mouse model according to the invention is associated with osteoinduction, a human homologue of said nucleic acid sequence or a corresponding human pathway is preferably determined. Such human homologue or pathway is a preferred target for influencing osteoinduction in humans.

Yet another application of a mouse model according to the present invention is the screening for substances that are capable of enhancing or counteracting heterotopic ossification. Now that a mouse model according to the invention has been provided, screenings at a larger scale, as compared to large animals such as dogs, have become possible wherein a plurality of substances are tested. In short, a scaffold with a known osteoinductive property is implanted subcutaneously into a population of a mouse model according to the invention. Subsequently, some mice are provided with test substances whereas at least one other mouse is not. After an incubation period, the extent of heterotopic bone formation in a reference mouse without any test substance (reference value) is compared to the extent of heterotopic bone formation in mice with a certain test substance. If the extent of heterotopic bone formation differs significantly between a reference mouse without test compound and a mouse with a certain test compound, it is concluded that the test compound is involved in osteoinduction. If the extent of heterotopic bone formation in a mouse with a certain test substance is significantly higher than the extent of heterotopic bone formation in the same kind of mouse without any test substance, it is concluded that the test substance is capable of enhancing heterotopic ossification. On the other hand, if the extent of heterotopic bone formation in a mouse with a certain test substance is significantly lower than the extent of heterotopic bone formation in the same kind of mouse without any test substance, it is concluded that the test substance is capable of counteracting heterotopic ossification. Efficient screening methods as described above have now become possible in view of the easy handling and breeding of mice, involving less facilities and significantly lower costs, as compared to large animals such as dogs and goats.

One aspect of the present invention therefore provides a use or method according to the invention, comprising determining whether a candidate substance is capable of enhancing heterotopic ossification. Further provided is a use or method according to the invention, comprising determining whether a candidate substance is capable of counteracting heterotopic ossification. The invention also provides a method for determining whether a test substance is capable of enhancing or counteracting osteoinduction, the method comprising:
- subcutaneously implanting an osteoinductive scaffold into a population of a mouse model according to the invention wherein the incidence of heterotopic bone-formation in the presence of a subcutaneous osteoinductive scaffold is at least 25%, preferably at least 30%, more preferably at least 40%, more preferably at least 50%, more preferably at least 60%, more preferably at least 70%, more preferably at least 80%, more preferably at least 90%, most preferably at least 95%;
- providing at least one mouse of said population with a test substance;
- incubating for at least 20 days, preferably at least 30 days, more preferably at least 40 days, more preferably at least 50 days, more preferably at least 60 days;
- determining the extent of heterotopic bone formation at the site of implantation in said at least mouse; and
- comparing said extent of heterotopic bone formation with the extent of heterotopic bone formation in a mouse of said population that has not been provided with said test substance, whereby a significantly higher extent of heterotopic bone formation in said mouse with said test substance as compared to the extent of heterotopic bone formation in said mouse without test substance indicates that the test substance is capable of enhancing heterotopic ossification and a significantly lower extent of heterotopic bone formation in said mouse with said test substance as compared to the extent of heterotopic bone formation in said mouse without test substance indicates that the test substance is capable of counteracting heterotopic ossification.

The above mentioned population comprises at least two mice. Preferably, however, said population comprises at least four mice, more preferably at least six mice, so that experiments wherein a test substance is investigated can be performed *in duplo or in triplo.* Said osteoinductive material is preferably a ceramic, more preferably a calcium phosphate ceramic since these materials have good osteoinductive properties. The osteoinductive scaffold is preferably implanted in a dorsal pocket, a leg or the abdomen of the mice of said population. Any method for administering a test compound to a mouse can be used in a method according to the invention. For instance, the test compound can be administered to the food or drink of the mouse, it can be injected intradermally, subcutaneously, intramuscularly or intravenously, it can be sprayed in the air in order to be inhaled, etc.

As shown in the Example, a mouse model according to the invention is particularly suitable for obtaining heterotopic bone. A mouse model of the invention is capable of forming heterotopic bone in the presence of a subcutaneously implanted osteoinductive scaffold, thereby avoiding ingrowth of surrounding bone tissue. Therefore, a use of a mouse model according to the invention for obtaining heterotopic bone is also provided herewith, as well as a mouse model, use or method according to the invention, wherein said mouse model comprises a subcutaneously implanted osteoinductive scaffold. Further provided is a method for inducing bone formation, the method comprising:
- subcutaneously implanting an osteoinductive material into a mouse model wherein the incidence of heterotopic bone-formation in the presence of a subcutaneous osteoinductive scaffold is at least 25%, and
- allowing the formation of heterotopic bone.

Again, said incidence of heterotopic bone-formation in the presence of a subcutaneous osteoinductive scaffold is preferably at least 30%, more preferably at least 40%, more preferably at least 50%, more preferably at least 60%, more preferably at least 70%, more preferably at least 80%, more preferably at least 90%, most preferably at least 95%. Said osteoinductive material is preferably a ceramic, more preferably a calcium phosphate ceramic since these materials are known for their good osteoinductive properties. In another preferred embodiment, said osteoinductive material is a polymer or a metal. The osteoinductive material is preferably implanted in a dorsal pocket, a leg or the abdomen of said mouse model.

After formation of heterotopic bone by a method according to the invention, at least part of the formed heterotopic bone is preferably harvested for further use, for instance for further research. This is for instance done by removing the implanted osteoinductive material with adhered heterotopic bone from the mouse.

A mouse model according to the present invention is characterized by the fact that the incidence of heterotopic bone-formation in the presence of a subcutaneously implanted osteoinductive scaffold is at least 25%. The Example shows two non-limiting strains of such mouse model. Accordingly, a mouse model according to the invention preferably comprises an FVB mouse or a 129 mouse. Of course, any substrain or related inbred strain or hybrid or progeny thereof is also particularly suitable, as long as they have maintained their incidence of heterotopic bone formation of at least 25%. Further provided is therefore a mouse model, use or method according to the invention, wherein said mouse model is an FVB mouse or a 129 mouse, or any substrain or related inbred strain or hybrid or progeny thereof.

An FVB mouse is defined herein as a mouse belonging to the FVB inbred strain. Likewise, a 129 mouse is defined herein as a mouse belonging to the 129 inbred strain. FVB mice are well known in the art. Derived in 1935 from an outbred Swiss colony at NIH, this strain was in the early 1970s established as an inbred strain. There after the Fvlb allele was inbred, which gave the strain its present name. FVB mice are particularly suitable for most transgenic experiments and genetic analyses, and they have large litters.

129 mice are also known in the art. They were developed at The Jackson Laboratory and introduced to the Pasteur Institute of Paris in the 1970s. 129 mice are particularly suitable for creating knockout mice and otherwise mutated mice.

As used herein, an inbred mouse strain is defined as a population of mice that has been obtained by mating brother x sister or parent x offspring for at least 20 consecutive generations. The individual mice of such population will on average have only 0.01 residual heterozygosity and are for most purposes regarded as genetically identical. A related inbred strain is defined herein as an inbred strain that has a common origin, but is separated before F20. The term " Fx" is used herein in its common definition, meaning the x^{th} generation offspring, wherein x is an integer that is greater than zero.

A substrain of a certain mouse strain is defined herein as a population of mice originating from a certain strain, wherein the substrain population has at least one genetic characteristic that is not shared by all members of the original strain. Substrains for instance arise by incomplete inbreeding at the time of separation from progenitors, generally by separating two branches after 20 but before 40 generations of inbreeding. Undetected spontaneous mutations that become fixed in a population (genetic drift) is also a cause of the arising of substrains, as well as deliberate outcrossing of strains for specific purposes. Separation of a subpopulation from its parent population for 20 or more generations also give rise to a substrain.

As used herein, a hybrid according to the invention is defined as the offspring resulting from the mating of a mouse model according to the invention with another mouse strain. Preferably, the other mouse is a homozygous mouse, such as a member of an inbred strain.

Preferred substrains of a 129 mouse model of the invention are 129S strains. More preferably, a 129S2 mouse model is provided and even more preferably a 129S2/Sv mouse model. In one preferred embodiment, a 129S2/SvPasCrl mouse model is provided. One aspect of the invention thus provides a mouse model, use or method according to the invention, wherein said mouse model is a 129S mouse, preferably a 129S2 mouse, more preferably a 129S2/Sv mouse, most preferably a 129S2/SvPasCrl mouse. In another particularly preferred embodiment a mouse model, use or method according to the invention is provided, wherein said mouse model is an FVB/N mouse, preferably a FVB/NCrl mouse. An FVB mouse is particularly preferred in view of its very high incidence of heterotopic bone formation.

The above mentioned strains and substrains are well known in the art. For instance, the 129S2/Sv substrain is derived from the 129S parental lineage (Sv means originally bred by Leroy Stevens). A 129S2/SvPasCrl mouse is a mouse from the 129S2/Sv substrain originating from the Pasteur Institute of Paris, now bred by Charles River Laboratories. A FVB/NCrl mouse is a mouse from the FVB/N strain bred by Charles River Laboratories.

It is to be understood that the invention is not limited to the above mentioned strains and substrains. Now that the invention has provided the insight that it is at all possible to use a mouse model for osteoinductive research, contrary to previous results in the art, it has become possible to develop other mouse models according to the invention. This is for instance done by crossing the strains or substrains described herein with other mice and screening the offspring for the desired incidence of heterotopic bone formation. Any population of mice can be screened for an incidence of heterotopic bone-formation in the presence of a subcutaneous osteoinductive scaffold of at least 25%.

As shown in the Example, a mouse model according to the invention can be chosen such that heterotopic bone is obtained with an average bone area / scaffold area of at least 1% after 12 weeks. This means that the area of *de novo* formed bone divided by the total area of the cross section of the implanted scaffold is at least 0.01. Such mouse model is preferred for uses and methods according to the invention. Even more preferably, a mouse model according to the invention is used which yields an average bone area / scaffold area of at least 1.5%, even more preferably at least 2%, and even more preferably at least 2.5% after 12 weeks. An FVB mouse model according to the invention has these properties and is therefore particularly preferred. This yield of heterotopic bone is higher than the amount of heterotopic bone obtained by Yuan et al, since Yuan et al obtained less than 1% (in only 3 out of 16 mice, the other mice not forming heterotopic bone at all). Hence, a mouse model according to the present invention not only has a much higher incidence of heterotopic bone formation, but can also be chosen such that a higher yield of heterotopic bone is obtained as compared to the art. One embodiment of the invention therefore provides a mouse model, use or method according to the invention, wherein said mouse model is able to form heterotopic bone in the presence of a subcutaneous osteoinductive scaffold with an average bone area / scaffold area of at least 1% after 12 weeks. A mouse model wherein at least such amount of heterotopic bone has been formed is also provided herewith. Such mouse model, containing heterotopic bone with an average bone area / scaffold area of at least 1%, is particularly useful for osteoinductive research as described herein before. For instance, the bone-containing mouse model is used for genetic analyses, for testing candidate scaffolds or for testing candidate substances for their capabilities of enhancing or counteracting heterotopic ossification. In one aspect the invention therefore provides a mouse model, use or method according to the invention, wherein said mouse model comprises heterotopic bone wherein the average bone area / scaffold area is higher than 1%. Preferably, the above mentioned mouse model comprises heterotopic bone wherein the average bone area / scaffold area is higher than 1.5%, even more preferably higher than 2.0%, most preferably higher than 2.5%.

The invention is further illustrated by the following Example. This Example is not limiting the invention in any way, but merely serves to clarify the invention.

### EXAMPLES

### Example 1

In this example we describe an *in vivo* study in which 10 different mice strains were screened for their osteogenic response to a previously identified osteoinductive material: tricalcium phosphate (TCP). We hypothesized that phenotypical differences between mice with different genetic background would determine their physiological response to TCP implanted subcutaneously. We have successfully identified two mice strains in which bone formation was induced by TCP.

### Materials and Methods

### Materials fabrication and sterilization

TCP porous ceramics were prepared from TCP powder (Plasma Biotal) and sintered at 1100 °C. Ceramic blocks (4×4×4 mm) were cut, cleaned ultrasonically with acetone, 70% ethanol and demineralized water, dried at 80 °C and autoclaved for sterilization. On half of the total amount of blocks, 5 µg of rh-BMP-2 was loaded per block by pipetting 25 µl of rh-BMP-2 in demi water (200 µg/ml) per block. TCP blocks loaded with rh-BMP-2 (TCPb) were left to vaccum dry in a sterile environment.

### Implantations in mice

TCP and TCP-b were implanted in 10 different commercially available mice strains: C3H/HeNHsd, DBA/1OlaHsd, DBA/2OlaHsd, CBA/CaOlaHsd, BALB/cOlaHsd, C57BL/6JOlaHsd (Harlan), FVB/NCrl, 129S2/SvPasCrl, SJL/JOrlCrl, CB17/Icr-*Prkdc^{scid}*/IcrCrl (Charles River). Preoperative pain relieve (Temgesic, Schering-Plough BV) was injected subcutaneously followed by general anesthesia consisting of a mix of isoflurane and oxygen. After shaving the back and disinfection of the skin with alcohol, small incisions were created on the dorsal sides. Pockets were opened in the incisions with blunt scissors. One block was inserted per pocket per mouse and all animals received both TCP and TCP-b. A total of 6 mice per strain were operated. Pockets were closed with sutures and animals allowed to recover from the anesthesia before returning to the cages. After 12 weeks, animals were euthanized with CO₂ inhalation and the implants retrieved.

### Histological processing

Explants were fixed in 1.5% glutaraldehyde / 0.14M cacodylate buffer. Afterwards they were dehydrated in a microwave with an ethanol/Milli-Q (% v/v) series and Milestone JFC solution (Milestone S.r.l.) in the following order: 70%, 80%, JFC and 100%. After dehydration, samples were left in methyl methacrylate (MMA) O/N at 4°C and the next day MMA was replaced by fresh solution. To polymerize, samples were left in a water bath at 37°C degrees. Tissue sections of approximately 10 to 15 µm thickness were obtained with a saw microtome (Leica SP 1600) and stained with Methylene Blue and Basic Fuchsin.

### Bone quantification

Tissue sections used for the quantification of bone were digitally scanned. Three non consecutive sections (when existent, otherwise consecutive) per sample per animal were quantified using Adobe Photoshop CS5: bone and scaffold areas were differentially pseudo-colored and the ratio between amount of pixels from each color converted to percentage of bone area per scaffold area. Some samples were excluded from the analysis because the implanted blocks were too dense and therefore not comparable with the majority of implanted porous ones. Statistical analysis was done with one-way ANOVA with Tukey's multiple comparison test.

### Results

### Bone induction by TCPb

Bone induction by TCPb was observed in all mice from the 10 different strains, indicating that all strains tested were capable of forming bone subcutaneously (positive control). In all cases, bone was observed both at the periphery of the implant (Figure 1 A and C) as well as in its porous spaces (Figure 1 B and D), not always in contact with the scaffold.

### Bone induction by TCP

Among the mice strains, two showed bone formation upon subcutaneous implantation of TCP: FVB/NCrl and 129S2/SvPasCrl. Regarding FVB/NCrl, bone was observed in all mice (6/6) whereas in 129S2/SvPasCrl the animal incidence was lower (4/5; one mouse died three weeks after surgery reducing the total amount of explants to 5). In both mice strains, bone tissue was mostly observed at the periphery of the implants (Figure 2 A and C), always in contact with the scaffold (Figure 2 B and D). TCP explants from FVB/NCrl showed an average bone area / scaffold area higher than that of 129S2/SvPasCrl, (approximately 2.8% compared to 0.2% respectively (Figure 2 E).

### Discussion

Here we report the induction of bone formation by TCP under the skin of FVB/NCrl and 129S2/SvPasCrl. The bone incidence and amounts are promising results, never seen before in the induction of bone in mice by subcutaneously implanted synthetic materials.

### References

Yang et al. Journal of Zhejiang University - Science B (Biomedicine & Biotechnology) 12(7), 2011: 582-590

Yuan et al. Tissue Engineering 12(6), 2006: 1607-1615

## Claims

1. A mouse model for investigating osteoinduction, comprising a subcutaneously implanted material, wherein the mouse model is **characterized in that** the incidence of heterotopic bone-formation in the presence of a subcutaneous osteoinductive scaffold is at least 25%.

2. Use of a mouse model according to claim 1 for investigating osteoinduction.

3. Use of a mouse model according to claim 1 for obtaining heterotopic bone, or for studying the osteoinductive capacity of a test material.

4. A method for inducing bone formation, the method comprising:
- subcutaneously implanting an osteoinductive material into a mouse model wherein the incidence of heterotopic bone-formation in the presence of a subcutaneous osteoinductive scaffold is at least 25%, and
- allowing the formation of heterotopic bone.

5. A method for determining whether a test material is capable of osteoinduction, the method comprising:
- subcutaneously implanting said test material into a mouse model wherein the incidence of heterotopic bone-formation in the presence of a subcutaneous osteoinductive scaffold is at least 25%, and
- determining whether heterotopic bone formation occurs at the site of implantation.

6. A method according to claim 5, further comprising selecting a material that has osteoinductive capacity.

7. A mouse model, use or method according to any one of claims 1-6, wherein said material is a ceramic.

8. A mouse model, use or method according to any one of claims 1-7, wherein said material is a calcium phosphate ceramic.

9. Use or method according to any one of claims 2-8, comprising determining whether a genomic nucleic acid sequence or a biological pathway of said mouse model is associated with osteoinduction.

10. Use or method according to any one of claims 2-9, comprising determining whether a candidate substance is capable of enhancing heterotopic ossification.

11. Use or method according to any one of claims 2-10, comprising determining whether a candidate substance is capable of counteracting heterotopic ossification.

12. A mouse model, use or method according to any one of claims 1-11, wherein said material is implanted in a dorsal pocket, a leg or the abdomen of said mouse.

13. A mouse model, use or method according to any one of claims 1-12, wherein said mouse model is able to form heterotopic bone in the presence of a subcutaneous osteoinductive scaffold, with an average bone area / scaffold area of at least 1% after 12 weeks.

14. A mouse model, use or method according to any one of claims 1-13, wherein said mouse model is an FVB mouse or a 129 mouse, or any substrain or related inbred strain or hybrid or progeny thereof.

15. A mouse model, use or method according to any one of claims 1-14, wherein said mouse model is an FVB/N mouse or a 129S mouse, preferably a 129S2 mouse.

16. A mouse model, use or method according to any one of claims 1-15, wherein said mouse model comprises a subcutaneously implanted osteoinductive scaffold.

17. A mouse model, use or method according to any one of claims 1-16, wherein said mouse model comprises heterotopic bone wherein the average bone area / scaffold area is higher than 1%.
